# EUROPEAN PATENT APPLICATION

(11) **EP 4 202 037 A1**
(43) Date of publication of application: **28.06.2023**
(21) Application number: 21217091.4
(22) Date of filing: 22.12.2021
(51) Int. Cl.: C12N 5/071, A01N 1/02, C12M 1/00

(54) **EX VIVO PRODUCTION OF COMPOUNDS OF INTEREST**

(71) Applicant: NEDERLANDSE ORGANISATIE VOOR TOEGEPAST- NATUURWETENSCHAPPELIJK ONDERZOEK TNO, 2595 DA 's-Gravenhage (NL)
(72) Inventor: VAN DE STEEG, Evita, 2595 DA 's-Gravenhage (NL); VAES, Wouter Henricus Johannes, 2595 DA 's-Gravenhage (NL); STEVENS, Lida Joanne, 2595 DA 's-Gravenhage (NL); ERPELINCK, Steven Leonardus Angela, 2595 DA 's-Gravenhage (NL)
(74) Representative: V.O.

(57) **Abstract**

The invention relates to methods for producing one or more compounds of interest, comprising *ex vivo* perfusing a liver or liver tissue and optionally a kidney or kidney tissue with a perfusion liquid comprising a precursor of said one or more compounds of interest, wherein said precursor is a compound that is metabolized in the liver and/or kidney, and purifying said one or more compounds of interest from the perfusate, bile, urine, perfused liver or liver tissue and/or perfused kidney or kidney tissue.

## Description

### Field of the invention

The invention relates to production of compounds of interest, in particular metabolites of precursors that are metabolized in the liver. In particular the invention relates to production of such compounds using *ex vivo* liver tissue or an *ex vivo* liver.

### Background of the invention

Upon oral intake of substances, including drugs, food ingredients and environmentally present pollution, intestinal absorption is the first barrier that affects the final drug concentration in the circulating blood. Following intestinal absorption, drugs reach the liver via the portal vein, where they can be absorbed, metabolized and/or excreted into the bile. The liver is responsible for the biotransformation of many endogenous and exogenous compounds and is involved in the storage and biliary excretion of these compounds and their metabolites. In addition to the liver, extrahepatic tissues such as the gastro-intestinal tract and the kidneys significantly contribute to the clearance of drugs, as a diverse range of transporters and metabolizing enzymes also have been characterized in these organs. Influx and efflux transporters (expressed on apical or basolateral membranes of these organs), and cytochrome P450 (CYP450) enzymes regulate the absorption of drugs across the intestinal wall into the portal blood, the clearance of drugs in the liver (and kidneys) and excretion by the biliary and renal pathways. The metabolites that are formed as a result of the metabolic breakdown of drugs can be toxic for the body and lead to side effects.

To predict the metabolism of candidate drugs a variety of preclinical models can be applied. *Ex vivo* tissue and organ models, including perfused whole organs, is an example of one of these preclinical models, see Stevens LJ. et al. (2020) for a review. Such methods make use of organs or organ tissue that are perfused with a perfusate to which the candidate drugs are added in physiologically relevant concentrations in order to mimic the *in vivo* pharmacokinetics following administration of the candidate drug. For instance, US10176887 describes a method of developing a pharmaceutical product, wherein a perfusate comprising at least one substance to be evaluated (i.e. the drug or drug candidates) is passed through at least one metabolically active human organ (e.g. through a liver) via a perfusion apparatus. Sample are taken at different time points and analyzed, e.g. absorption, transport, metabolism, elimination, efficacy and/or toxicity are determined biochemically by assaying for toxic metabolites or end-products. The data is used to determine whether to continue with the development of the substance further into a pharmaceutical product. The methods aim to provide a drug developer a tool to test compounds in an environment representative of the human condition.

In order to study the side effects of a drug, including its metabolites, in preclinical model and in particular in clinical studies, reference standards of known drug metabolites are used. This requires the production of such reference metabolites at a sufficiently large scale. The production of such metabolites at scale larger than micrograms is currently almost exclusively done by chemical synthesis, which processes are both costly and time consuming.

Hence, there remains a need in the art for improved methods to produce reference standard metabolites.

### Summary of the invention

It is an object of the present invention to provide methods to produce compounds of interest, in particular drug metabolites, that overcome one or more of the advantages described above.

The invention therefore provides a method for producing one or more compounds of interest, the method comprising:
- *ex vivo* perfusing a liver or liver tissue and optionally a kidney or kidney tissue with a perfusion liquid comprising a precursor of said one or more compounds of interest, wherein said precursor is a compound that is metabolized in the liver and/or kidney, and
- purifying said one or more compounds of interest from the perfusate, bile, urine, perfused liver or liver tissue and/or perfused kidney or kidney tissue.

In another aspect, the invention provides a method for obtaining one or more compounds of interest, the method comprising:
- *ex vivo* perfusing a liver or liver tissue and optionally a kidney or kidney tissue with a perfusion liquid comprising a precursor of said one or more compounds of interest, wherein said precursor is a compound that is metabolized in the liver and/or kidney, and
- purifying said one or more compounds of interest from the perfusate, bile, urine, perfused liver or liver tissue and/or perfused kidney or kidney tissue.

In preferred embodiments, a method of the invention comprises isolating and purifying the one or more compounds of interest from the perfusate, bile, urine and/or perfused liver or liver tissue.

In preferred embodiments, the liver or liver tissue is continuously perfused with said perfusion liquid.

In preferred embodiments, the liver or liver tissue comprises a portal vein and a hepatic artery and optionally the kidney or kidney tissue comprises a renal artery, and the liver or liver tissue is perfused through the portal vein and the hepatic artery, and optionally the kidney or kidney tissue which is perfused through the renal artery.

In preferred embodiments, the perfusion liquid comprises at least 10 mg, preferable at least 100 mg of said precursor.

In preferred embodiments, the perfusion is performed under normothermic or subnormothermic conditions.

In preferred embodiments, the perfusion liquid is continuously oxygenated.

In preferred embodiments, the perfusion liquid comprises red blood cells and plasma.

In preferred embodiments, the perfusion liquid comprises one or more compounds selected from the group consisting of bile acid, such as cholate or taurocholate, insulin, epoprostenol, heparin, amino acids, vitamins and glucose .

In preferred embodiments, the liver or liver tissue is perfused for at least 4 hours, preferably for 4 to 24 hours.

In preferred embodiments, the liver or liver tissue is comprised in an organ system further comprising a gall bladder or gall bladder tissue and the one or more compounds of interest are further purified from bile, preferably isolated and purified from bile, and/or the liver or liver tissue is comprised in an organ system further comprising a kidney or kidney tissue and the and one or more compounds of interest are further purified from urine, preferably isolated and purified from urine.

In preferred embodiments, the liver or liver tissue and kidney or kidney tissue are perfused in series, preferably wherein the liver or livers tissue is first perfused with the perfusion liquid comprising a precursor of said one or more compounds of interest and the kidney or kidney tissue is perfused with the perfusate from the perfused liver or liver tissue.

In preferred embodiments, the liver or liver tissue is a porcine liver or porcine liver tissue and optionally the gall bladder or gall bladder tissue is a porcine gall bladder or porcine gall bladder tissue and/or the kidney or kidney tissue is a porcine kidney or porcine kidney tissue.

In preferred embodiments, purifying comprises one or more selected from the group consisting of extraction, including differential extraction, crystallization, distillation, including simple distillation, fractional distillation and steam distillation, and chromatography, including adsorption chromatography, column chromatography, thin layer chromatography (TLC), partition chromatography and combinations thereof, preferably wherein the purifying comprises extraction using an organic solvent.

In preferred embodiments, a method of the invention further comprises packaging the one or more purified compounds of interest.

### Detailed description

As used herein, "to comprise" and its conjugations is used in its non-limiting sense to mean that items following the word are included, but items not specifically mentioned are not excluded. In addition the verb "to consist" may be replaced by "to consist essentially of" meaning that a compound or adjunct compound as defined herein may comprise additional component(s) than the ones specifically identified, said additional component(s) not altering the unique characteristic of the invention.

The articles "a" and "an" are used herein to refer to one or to more than one (i.e., to at least one) of the grammatical object of the article. By way of example, "an element" means one element or more than one element.

The word "approximately" or "about" when used in association with a numerical value (approximately 10, about 10) preferably means that the value may be the given value of 10, plus or minus 10% of the value, preferably plus or minus 5% of the value, more preferably plus or minus 1% of the value.

The use of the alternative (e.g., "or") should be understood to mean either one, both, or any combination thereof of the alternatives.

As used herein the term "hepatic metabolism" refers to chemical alterations to compounds such as drugs that occur in the liver, in particular by enzymatic activity. These compounds are processed by a variety of soluble and membrane-bound enzymes, especially those related to the hepatocyte endoplasmic reticulum. Each drug has its specific enzyme disposal pathway(s) of biotransformation involving one or more of these enzyme systems. Drug metabolism is achieved through phase I reactions, phase II reactions, or both phase I and phase II reactions. Phase I reactions of drug metabolism involve oxidation, reduction, or hydrolysis of a compound, resulting in its conversion to a more polar molecule increasing solubility. The cytochrome P450 (CYP) system is considered to be the most important enzyme in Phase I hepatic metabolism. Phase II reactions involve conjugation by coupling the compound or its metabolites resulting from Phase I metabolism to another molecule, such as acetylation, glucuronidation, sulfation and methylation.

As used herein, the term "metabolically active" in the context of an organ or organ tissue refers to having the metabolic activity *ex vivo* that is characteristic of the living organism from which the organ or organ tissue is derived.

As used herein, the term "toxicity" encompasses physical, chemical, biochemical and biological damage to tissue or organs. Toxicity encompasses any deleterious effect on tissues and organs, such as, but not limited to cell death, apoptosis, necrosis, genetic mutation, changes in gene expression, biochemical inhibition, and oxidative damage.

As used herein, the term "metabolically active" refers to having the metabolic activity characteristic of the living organism from which the organ or organ tissue is derived.

As used herein, the term "liquid" and "perfusion liquid" are used interchangeably and refer to the liquid that is used to perfuse the liver or liver tissue and optionally further organs or organ tissue in a method of the invention. The precursor of interest is present in or added to the liquid or perfusion liquid.

As used herein, the term "perfusate" refers to the liquid of perfusion liquid after perfusion of the liver or liver tissue and optionally further organs or organ tissue. The perfusate may comprise the one or more compounds of interest.

As used herein, the term "hypothermic", such as in the context of hypothermic perfusion, hypothermic perfusion conditions or hypothermic perfusion liquid, refers to a temperature below normal body temperature of the species from which the liver, liver tissue, and optionally further organ or organ tissue, is derived. Preferably, "hypothermic" refers to a temperature of between 0 and 22 °C, more preferably of between 1 and 18 °C, more preferably between 1 and 10 °C.

As used herein, the term "normothermic", such as in the context of normothermic perfusion, normothermic perfusion conditions or normothermic perfusion liquid, refers to a temperature that is similar to normal body temperature of the species from which the liver, liver tissue, and optionally further organ or organ tissue, is derived. Preferably, "normothermic" refers to a temperature of between 36 and 38 °C, more preferably of between 36.5 and 37.5 °C, more preferably about 37 °C.

As used herein, the term "subnormothermic", such as in the context of subnormothermic perfusion, subnormothermic perfusion conditions or subnormothermic perfusion liquid, refers to a temperature that is below normal body temperature of the species from which the liver, liver tissue, and optionally further organ or organ tissue, is derived, but preferably above 20 °C. Preferably, "subnormothermic" refers to a temperature of between 20 and 25 °C.

The present inventors have developed novel methods to produce metabolites of compounds, such as pharmaceutical compounds, that are metabolized in the liver. The methods make use of *ex vivo* liver or liver tissue that is perfused with a liquid containing the precursor of the metabolites. It has been found that it is possible to perfuse porcine liver with relatively high concentrations of pharmaceutical compounds in a relatively short period of time to obtain high amounts of the desired metabolites. As demonstrated in the examples a method of invention allows to produce in total 3.5 mg midazolam 1'O-glucuronide from 40 mg midazolam, whereby 1.5 mg midazolam 1'O-glucuronide is obtained from plasma and bile and 2 mg midazolam 1'O-glucuronide is obtained from liver tissue. It was further shown that a method of the invention allows to produce several metabolites from atorvastatin.

This finding is not tied to a particular compound, but can be used for any compound of interest that is formed by hepatic metabolism. The examples demonstrate the production of metabolites of midazolam and atorvastatin in sufficiently high quantities, but it is emphasized that the present invention is applicable to various compounds, including drugs, and hence applicable in the production of all kinds of liver metabolites. As will be appreciated, the exact amount of a particular compound may need to be determined based on the properties of the compound, in particular liver toxicity and optionally kidney toxicity.

In a first aspect the invention provides a method for producing one or more compounds of interest, the method comprising:
- *ex vivo* perfusing a liver or liver tissue and optionally a kidney or kidney tissue with a perfusion liquid comprising a precursor of said one or more compounds of interest, wherein said precursor is a compound that is metabolized in the liver and/or kidney, and
- purifying said one or more compounds of interest from the perfusate, bile, urine, perfused liver or liver tissue and/or perfused kidney or kidney tissue.

In a method of the invention one or more compounds of interest are produced. In one embodiment a method for producing one or more compounds of interest is a method for obtaining one or more compounds of interest. In one embodiment a method for producing one or more compounds of interest is a method for perfusing a liver or liver tissue. The one or more compounds are preferably metabolites of a precursor of said one or more compounds of interest. I.e. the one or more compounds are preferably formed by hepatic metabolism and/or renal metabolism. Preferably, if more than one compound of interest are produced or obtained, these are metabolites of the same precursor. In preferred embodiments, the one or more compounds of interest are metabolites of a precursor that are formed by hepatic metabolism, preferably in the human and/or porcine liver.

As used herein, the term "precursor" refers to a compound from which another compound is formed, in particular in a metabolic reaction, preferably a metabolic reaction in the human body. In a method of the invention a precursor is compound that is metabolized in the liver and/or kidney, in particular in the human liver and/or kidney, more preferably in the human and porcine liver and/or kidney. In one embodiment, the precursor and/or the compound of interest is a pharmaceutical compound.

Compounds that are metabolized in the liver and their metabolites are well known. A method of the invention is in particular useful for producing compounds of interest that are known to be the subject of hepatic and/or renal metabolism. In the examples the production of two structurally very different compounds, i.e. midazolam 1'O-glucuronide from precursor midazolam and atorvastatin lactone, 2-hydroxy atorvastatin, 2-hydroxy atorvastatin lactone, 4-hydroxy atorvastatin and 4-hydroxy atorvastatin lactone from precursor atorvastatin, is demonstrated. Indeed, as described herein above, a method of the invention can be used to produce and obtain any compound of interest that is formed by hepatic and/or renal metabolism.

A method of the invention comprises *ex vivo* perfusing a liver or liver tissue with a liquid comprising a precursor of one or more compounds of interest. In a preferred embodiment a liver is perfused.

The liver is preferably a healthy liver and the liver tissue is preferably healthy liver tissue. As used herein "healthy" refers to the absence of known disease or infection.

In preferred embodiments, the liver or liver tissue is metabolically active.

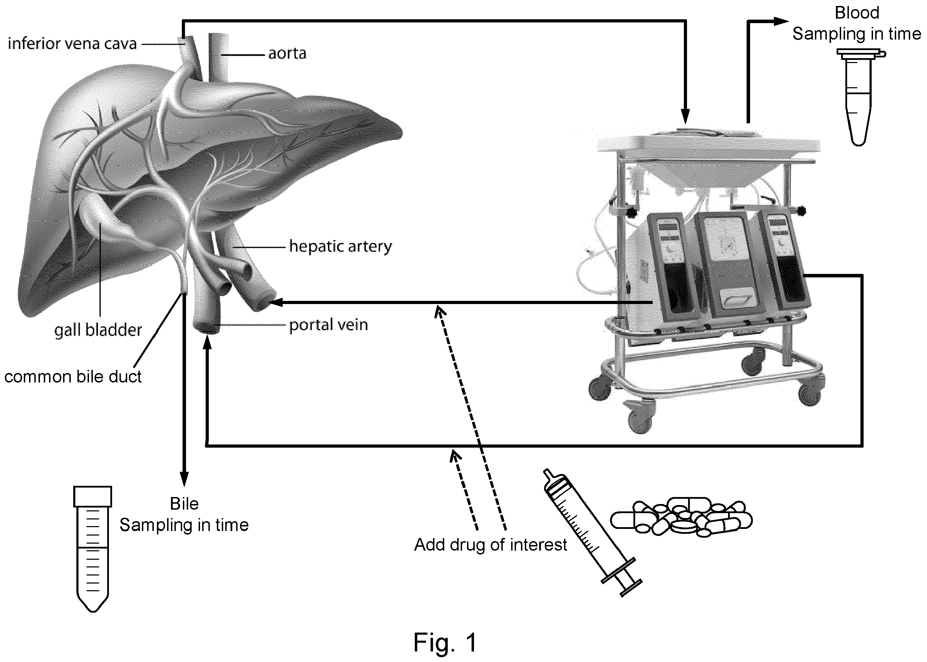

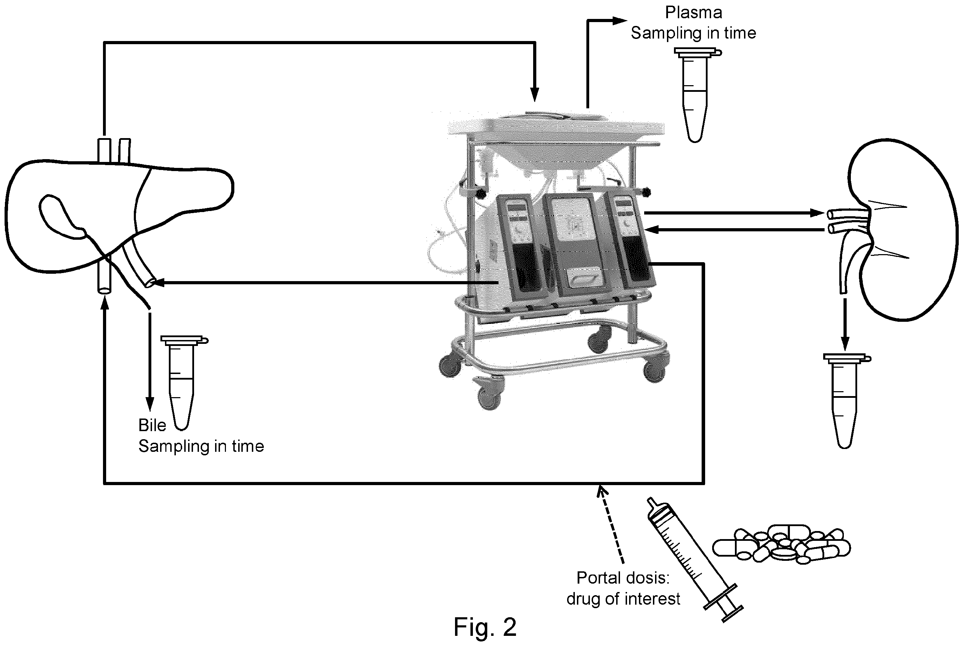

In preferred embodiments, the liver or liver tissue comprises a portal vein or part thereof that supplies the liver or liver tissue with the perfusion liquid and/or a hepatic artery or part thereof that supplies the liver or liver tissue with the perfusion liquid, preferably both a portal vein or part thereof and a hepatic artery or part thereof. Reference is made to figures 1 and 2, showing exemplary organ systems with (part of) the portal vein and (part of) the hepatic artery. In a method of the invention, the liver or liver tissue is preferably perfused through the portal vein and/or the hepatic artery or said parts thereof. Preferably, the liver or liver tissue is perfused through the portal vein and the hepatic artery or said parts thereof. In preferred embodiments, the liver or liver tissue, preferably liver, comprises an inferior vena cava or part thereof from which the perfusate can be collected and/or an aorta or part thereof from which the perfusate can be collected. Preferably, the liver or liver tissue, preferably liver, comprises an inferior vena cava or said part thereof and an aorta or said part thereof.

In one preferred embodiment, the liver or liver tissue, preferably liver, is comprised in an organ system. As used herein the term "organ system" refers to the combination of all organs or organ tissues that are perfused in method of the invention. All organs in the organ system are preferably healthy organs and all organ tissue in the organ system is preferably healthy organ tissue.

In one embodiment, a liver or liver tissue is the sole organ or organ tissue present in an organ system used in a method of the invention. However, metabolism occurs in many organs in the body, including the liver, intestinal wall, lungs, kidneys, and in plasma. Hence, an organ system may comprise one or more further organs or organ tissue, in particular organ or organ tissue that is further perfused with the perfusion liquid comprising the precursor of the one or more compounds of interest. Preferred tissues and organs include, but are not limited to, gall bladder, kidney, intestine, pancreas, heart, testes, placenta, thymus, adrenal gland, lymph nodes and tissues thereof.

In a preferred embodiment, the organ system comprises a gall bladder or gall bladder tissue, preferably a gall bladder, in addition to the liver or liver tissue. Figure 1 depicts an example of such organ system. In preferred embodiments, the gall bladder or gall bladder tissue comprises a common bile duct or part thereof. Preferably, the cystic duct is ligated and the common bile duct is cannulated. Using such organ system, the one or more compounds of interest are further preferably purified from bile, preferably isolated and purified from bile, in particular collected from the common bile duct or part thereof. In one embodiment, the liver or liver tissue and the gall bladder or gall bladder tissue are the sole organs or organ tissue present in an organ system used in a method of the invention.

In another preferred embodiment, the organ system comprises a kidney or kidney tissue, preferably a kidney, in addition to the liver or liver tissue and the kidney or kidney tissue, preferably kidney, is further perfused with perfusion liquid. Figure 1 depicts an example of such organ system. In preferred embodiments, the kidney or kidney tissue comprises a renal artery or part thereof that supplies the kidney or kidney tissue with the perfusion liquid. In some embodiments, the kidney or kidney tissue comprises a renal vein or part thereof. In preferred embodiments, the kidney or kidney tissue comprises a ureter or part thereof. Using such organ system, the one or more compounds of interest are further preferably purified from urine, preferably isolated and purified from urine. In one embodiment, the liver or liver tissue and the kidney or kidney tissue are the sole organs or organ tissue present in an organ system used in a method of the invention.

In a preferred embodiment, the organ system comprises, in addition to the liver or liver tissue, both a gall bladder or gall bladder tissue, preferably a gall bladder and preferably comprising a common bile duct or part thereof as described herein above, and a kidney or kidney tissue, preferably a kidney and preferably comprising a renal vein or part thereof and/or renal artery or part thereof and/or ureter or part thereof as described herein above, and the kidney or kidney tissue, preferably kidney, is further perfused with perfusion liquid. In one embodiment, the liver or liver tissue, the gall bladder or gall bladder tissue and the kidney or kidney tissue are the sole organs or organ tissue present in an organ system used in a method of the invention.

The organs and/or organ tissues in an organ system may or may not be connected. The different organs or organ tissues may be perfused in parallel or sequentially, or a combination thereof. In sequential perfusion, for instance the liver or liver tissue is first perfused with perfusion liquid and a further organ or organ tissue such as a kidney of kidney tissue is at least in part perfused with the perfusate of the liver. Preferably in sequential perfusion, for instance the liver or liver tissue is first perfused with perfusion liquid via the portal vein and a further organ or organ tissue such as a kidney of kidney tissue is perfused with the perfusate of the liver, and the arterial perfusion supplies the renal artery as well as the hepatic artery via a Y-connection. In parallel perfusion, the different organs are all perfused separately with perfusion liquid, which may be combined after perfusion. In parallel perfusion the arterial perfusion is preferably split to the hepatic artery and e.g. the renal artery, e.g. via a Y-connection, and preferably portal perfusion is to the portal vein of the liver.

In a preferred embodiment, the organ system comprises a liver or liver tissue and a kidney or kidney tissue and the liver or liver tissue and kidney or kidney tissue are perfused in parallel and the perfusion liquid obtained from the organs and/or tissue is optionally combined. In parallel perfusion the arterial perfusion is preferably split to the hepatic artery and e.g. the renal artery, e.g. via a Y-connection, and preferably portal perfusion is to the portal vein of the liver.

In another embodiment, the organ system comprises a liver or liver tissue and a kidney or kidney tissue and the liver or liver tissue and kidney or kidney tissue are perfused in sequentially, preferably wherein the liver or livers tissue is first perfused with the perfusion liquid comprising a precursor of said one or more compounds of interest and the kidney or kidney tissue is perfused with the perfusate from the perfused liver or liver tissue.

In alternative embodiments, the different organs or organ tissues comprised in an organ system as defined herein are not connected and are perfused separately, i.e. with different batches of perfusion liquid.

The liver or liver tissue and optionally further organ or organ tissue comprised in an organ system used in a method of the invention can be of any species that is capable of producing the compound of interest from the precursor by hepatic metabolism.

In one embodiment, the liver or liver tissue is human liver or liver tissue and other organ or organ tissue, such as gall bladder or gall bladder tissue and/or kidney or kidney tissue, is human organ or human organ tissue, e.g. donated human liver or other organ.

In one embodiment, the liver or liver tissue, and optionally further organ or organ tissue comprised in an organ system used in a method of the invention, is from a non-human animal. Such liver or liver tissue and optionally other organ or organ tissue is for instance obtained from a slaughterhouse.

In preferred embodiments, the liver or liver tissue, and optionally further organ or organ tissue comprised in an organ system used in a method of the invention, is from an animal of the genus Sus, preferably *Sus domesticus.* Hence, in a particularly preferred embodiment the liver or liver tissue is a porcine liver or porcine liver tissue, and optionally further organs or organ tissue comprised in an organ system used in a method of the invention are porcine organs or organ tissue. If present, the gall bladder or gall bladder tissue is preferably a porcine gall bladder or porcine gall bladder tissue and/or the kidney or kidney tissue is a porcine kidney or porcine kidney tissue. As demonstrated in the examples, the porcine and human liver have comparable transporter and enzyme expression.

The perfusion liquid that is used to perfuse the liver or liver tissue, preferably liver, and optionally further organ or organ tissue comprised in an organ system used in a method of the invention, comprises a precursor of the one or more compounds of interest. It may comprise more than one different precursors of the one or more compounds of interest. The perfusion liquid is preferably a normothermic perfusion liquid.

The perfusion liquid, preferably for normothermic perfusion, is preferably an aqueous solution, preferably a buffered aqueous solution. The perfusion liquid preferably has a pH of between 7 and 7.8, more preferably between 7.3 and 7.5, more preferably 7.4.

In a preferred embodiment, the perfusion liquid comprises red blood cells and/or plasma or albumin, preferably red blood cells and plasma or red blood cells and albumin, e.g. bovine serum albumin (BSA). In preferred embodiments, the perfusion liquid comprises between 40 and 60% red blood cells and between 60 and 40% plasma, more preferably about 50% red blood cells and about 50% plasma. In preferred embodiments, the red blood cells and/or plasma are porcine red blood cells and plasma, in particular if the organ or organ tissue that is perfused is porcine organ or porcine organ tissue.

In a preferred embodiment, the perfusion liquid used to perfuse the liver or liver tissue and optionally kidney or kidney tissue comprises one or more compounds selected from the group consisting of bile acid, such as cholate or taurocholate, insulin, epoprostenol, heparin, amino acids, vitamins and glucose. In preferred embodiments, the perfusion liquid used to perfuse the liver or liver tissue and optionally kidney or kidney tissue comprises bile acid, such as cholate or taurocholate, insulin, epoprostenol, and heparin. In preferred embodiments, the perfusion liquid used to perfuse the liver or liver tissue and kidney or kidney tissue, preferably kidney, comprises bile acid, such as cholate or taurocholate, insulin, epoprostenol, heparin, amino acids, vitamins and glucose. These compounds may be present in the perfusion liquid. Alternatively, the liver or liver tissue and optionally further organ or organ tissue comprised in an organ system used in a method of the invention perfusion liquid is continuously perfused with a perfusion liquid comprising e.g. red blood cells and plasma and at a certain time point after perfusion has started, the one or more compounds are added to the perfusion liquid, e.g. by injection, preferably continuous injection. An example of a suitable perfusion liquid is depicted in table 2.

The perfusion liquid comprises a precursor of the one or more compounds of interest. The maximal amount of the precursor that is perfused with a method of the invention depends on the characteristics of the precursor, in particular the degree of metabolization, liver toxicity and potentially toxicity for other organs or tissue that are comprised in an organ system that is used in a method of the invention.

In general up to several grams of precursor can be perfused with a method of the invention in a single perfusion. As used herein, the term "single perfusion" refers to a preferably continuous perfusion with a method of the invention during a certain period of time, such as e.g. 4 hours, 6 hours or 8 hours, during which the perfusion system maintains in place. In preferred embodiments at least 10 mg of the precursor is perfused with a method of the invention in a single perfusion, more preferably at least 25 mg of the precursor, more preferably at least 50 mg of the precursor, more preferably at least 100 mg of the precursor, more preferably at least 200 mg of the precursor, more preferably at least 300 mg of the precursor, more preferably at least 400 mg of the precursor, more preferably at least 500 mg of the precursor, more preferably at least 600 mg of the precursor, more preferably at least 700 mg of the precursor, more preferably at least 800 mg of the precursor, more preferably at least 900 mg of the precursor, such as between 100 mg and 3 gram of the precursor, between 200 mg and 3 gram of the precursor, between 300 and 3 gram of the precursor, between 400 mg and 2 gram of the precursor, between 500 mg and 1 gram of the precursor, or between 600 mg and 1 gram of the precursor. In some embodiments at least 1 gram of precursor is perfused with a method of the invention in a single perfusion.

Hence, in a preferred embodiment, the perfusion liquid comprises at least 10 mg of the precursor, more preferably at least 25 mg of the precursor, more preferably at least 50 mg of the precursor, more preferably at least 100 mg of the precursor, more preferably at least 200 mg of the precursor, more preferably at least 300 mg of the precursor, more preferably at least 400 mg of the precursor, more preferably at least 500 mg of the precursor, more preferably at least 600 mg of the precursor, more preferably at least 700 mg of the precursor, more preferably at least 800 mg of the precursor, more preferably at least 900 mg of the precursor, , such as between 100 and 1 gram of the precursor, between 200 mg and 3 gram of the precursor, between 300 mg and 3 gram of the precursor, between 400 mg and 2 gram of the precursor, between 500 mg and 1 gram of the precursor, or between 600 mg and 1 gram of the precursor. In some embodiments the perfusion liquid comprises at least 1 gram of the precursor.

The amount of perfusion liquid that is perfused through the liver or liver tissue and optionally further organ or organ tissue comprised in an organ system used in a method of the invention, may vary and is preferably between 1 and 4 litres, such between 1 and 3 litres, between 1.5 and 2.5 litres or about 2 litres. The total amount of the precursor is contained within this amount or perfusion liquid.

The liver or liver tissue and optionally further organ or organ tissue comprised in an organ system used in a method of the invention, may be perfused with a perfusion liquid under hypothermic conditions to re-charge the liver and/or optimise the energy status of the liver, liver tissue, organ or organ tissue until perfusion with the precursor compound. This will aid in maintaining the liver or liver tissue and optionally further organ or organ tissue in metabolically active state. Hypothermic preservation liquids are well known in the art and a skilled person is well capable of selecting an appropriate hypothermic preservation liquid for the relevant organ or organ tissue.

The liver or liver tissue, preferably liver, is preferably removed from e.g. hypothermic preservation or perfusate and, preferably, the portal vein and/or hepatic artery, are cannulated. Subsequently, and if necessary, the liver or liver tissue and optionally other organ or organ tissue can be flushed to remove e.g. hypothermic perfusion liquid or preservation solution

In some embodiments, the liver or liver tissue and optionally further organ or organ tissue comprised in an organ system used in a method of the invention is perfused first with a perfusion liquid that does not comprise the precursor, followed by perfusion with a perfusion liquid comprising the precursor. The perfusion liquids can be the same, except for the presence or absence of the precursor, or different. It is possible to replace the perfusion liquid without precursor by perfusion liquid without precursor. Alternatively, the precursor is added to the perfusion liquid during perfusion. In a preferred embodiment, the liver or liver tissue and optionally further organ or organ tissue comprised in an organ system used in a method of the invention perfusion liquid is continuously perfused with the perfusion liquid and at a certain time point after perfusion has started, the precursor is added to the perfusion liquid, e.g. by injection, preferably continuous injection. This continuous injection can for instance be a slow bolus, wherein the precursor is continuously added during a limited period of time, such as 5, 10 or 15 minutes. Alternatively, the precursor is added continuously during a more extended period of time such as 1 - 8 hours. In one embodiment the precursor is added continuously with a fixed concentration. In another embodiment, the precursor is added at an increasing, a decreasing or a variable concentration.

In preferred embodiments the perfusion is performed under normothermic or subnormothermic conditions. In further preferred embodiments the perfusion is performed under normothermic conditions.

In preferred embodiments, the perfusion liquid is continuously oxygenated.

Preferably, the liver or liver tissue, and optionally further organ or organ tissue comprised in an organ system used in a method of the invention, is/are perfused under physiological temperature, pressure, oxygenation, osmolality, electrolyte balance and pH.

In preferred embodiments the perfusion of the liver or liver tissue, preferably liver, is a dual perfusion, i.e. perfusion via both the portal vein and the hepatic artery. In preferred embodiments, the pressure of perfusion through the portal vein is between 8 and 11 mmHg, preferably about 11 mmHg. In preferred embodiments, the pressure of perfusion through the hepatic artery is between 50 and 80 mmHg, preferably about 50 mmHg.

If an organ system is used in a method of the invention comprising also a kidney or kidney tissue, preferably a kidney, the perfusion of the kidney or kidney tissue, preferably kidney, is perfusion via the renal artery. In preferred embodiments, the pressure of perfusion through the renal artery vein is between 70 and 95 mmHg, preferably about 85 mmHg.

The liver or liver tissue, and optionally further organ or organ tissue comprised in an organ system used in a method of the invention, may be perfused for a long as is necessary to obtain the required amount of the one or more compounds or interest. In preferred embodiments, liver or liver tissue and optionally further organ or organ tissue comprised in an organ system used in a method of the invention is/are perfused for at least 4 hours, preferably for 4 to 24 hours, such as 4 to 16 hours, 4 to 12 hours, 4 hours, 5 hours, 6 hours, 7 hours, 8 hours, 9 hours or 10 hours. In some embodiments, liver or liver tissue and optionally further organ or organ tissue comprised in an organ system used in a method of the invention is/are perfused for between 4 and 8 hours.

The amount of perfusion liquid used in a single perfusion may depend on the particular precursor, in particular its characteristics such as the degree of metabolization and liver toxicity. For instance, if a particular precursor has a relatively high liver toxicity, the concentration of precursor is relatively moderate and a higher amount of perfusion liquid may be required to obtain sufficient quantity of the one or more compounds of interest. The amount of perfusion liquid that is perfused through the liver or liver tissue and optionally further organ or organ tissue comprised in an organ system used in a method of the invention, may vary and is preferably between 1 and 4 litres, such between 1 and 3 litres, between 1.5 and 2.5 litres or about 2 litres.

In a preferred embodiment, the liver or liver tissue, and optionally further organ or organ tissue comprised in an organ system used in a method of the invention, is continuously perfused with the perfusion liquid comprising the precursor. This has the advantage that the organs or organ tissue is provided with a constant concentration of the precursor, which aids in achieving a high yield as possible.

During perfusion in a method of the invention several measurement can be performed to monitor e.g. progression of the perfusion, formation of the one or more compounds of interest and condition of the organs, organ tissue or organ system. For instance, perfusate and bile samples can be taken at regular intervals during perfusion, as well as liver biopsies. Parameters such as pH, lactate, pO2, flow parameters, bile production, etc. can be measured at regular intervals during perfusion. Methods to measure such parameters are standard in the art and can be readily performed by a person skilled in the art.

Any perfusion system can be used in a method of the invention. A skilled person is well capable of selecting an appropriate perfusion system. Non-limiting examples of suitable perfusion systems are LiverAssist ( XVIVO), OrganOx Metra (OrganOx) and Lifeport liver transporter (Organ Recovery Systems).

A method of the invention preferably comprise collecting the perfusate and/or the perfused liver or liver tissue, preferably collecting both the perfusate and the perfused liver or liver tissue. If the liver or liver tissue is comprised in an organ system further comprising a gall bladder or gall bladder tissue, bile is preferably further collected. If the liver or liver tissue is comprised in an organ system further comprising a kidney or kidney tissue, urine and/or kidney or kidney tissue is preferably further collected. In a preferred embodiment, the liver or liver tissue is comprised in an organ system further comprising a gall bladder or gall bladder tissue and a kidney or kidney tissues. In such embodiment, the perfusate, liver or liver tissue, bile and/or urine are collected. Preferably, the perfusate, liver or liver tissue, bile and urine are collected.

A method of the invention comprises purifying the one or more compounds of interest from the perfusate, perfused liver or liver tissue and/or bile. Optionally, one or more compounds of interest are purified from other organ or organ tissue comprised in an organ system that is perfused with a method of the invention and/or from urine.

In preferred embodiments, the one or more compounds of interest are isolated and purified from the perfusate, perfused liver or liver tissue, bile, urine and/or other organ or organ tissue. In particular preferred embodiments, the one or more compounds of interest are isolated and purified from the perfusate, perfused liver or liver tissue and bile. Optionally, one or more compounds of interest are isolated and purified from other organ or organ tissue comprised in an organ system that is perfused with a method of the invention and/or from urine if the organ system comprises a kidney and/or kidney tissue.

Isolation and purification of the one or more compounds of interest can be performed by any method suitable for isolating and purifying the particular compound or interest. In some embodiments, isolation and purification are performed in a single step. As used herein purification refers to separation of one or more compounds of interest from other components of the perfusate, liver or liver tissue, bile, urine and/or other organ or organ tissue. In preferred embodiment, purification refers to separation of one compound of interest from other components of the perfusate, liver tissue or bile. If more than one compound of interest are produced with a method of the invention, it is preferred, but not necessary, that each compounds of interest is purified, i.e. separated from other components of the perfusate, liver tissue, bile, urine and/or other organ or organ tissue and from other compounds of interest. Conventional methods can be used for isolation and purification, such as extraction, including differential extraction, crystallization, distillation, including simple distillation, fractional distillation and steam distillation, and chromatography, including adsorption chromatography, column chromatography, thin layer chromatography (TLC) and partition chromatography. In preferred embodiments, said purification comprises extraction using an organic solvent such as dimethylformamide, dimethyl sulfoxide, tetrahydrofuran, acetone, toluene and the like.

As described herein above and demonstrated in the examples, the present inventors have shown that high yields of the compounds of interest can be obtained with a method of the invention. In particular, the yield is in the mg range.

In preferred embodiments, purified compounds are obtained. As used herein, "purified compound" means that the obtained, product contains at least 90%, preferably at least 95%, more preferably at least 98%, more preferably at least 99% of a single compound of interest by weight of the product.

In preferred embodiments, a method of the invention further comprises packaging the one or more purified compounds, in particular packaging the one or more purified compounds in a suitable container. The total amount of a purified compound of interest obtained in a method of the invention can be packaged in a single container. Alternatively, aliquots or a purified compound of interest are packaged into separate containers.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

Figure 1: Exemplary organ system comprising a liver, gall bladder, portal vein and hepatic artery for use in a method of the invention.
Figure 2: Exemplary organ system comprising a liver, gall bladder, kidney, portal vein and hepatic artery for use in a method of the invention.
Figure 3: Comparable transporter & enzyme expression in human and porcine liver. Absolute expression (fmol/mg tissue) of various uptake and efflux transporter proteins within the plasma membrane of porcine livers (n=5) compared to the protein expression in human liver (n=15). Significant differences are denoted as asterisks (Students t-test *P<0.05, **P<0.01, ***P<0.001).
Figure 4: Pharmacokinetic profile of atorvastatin (dosed as slow bolus of 0.104 mg over 10 minutes to portal vein) in perfusate (A) and bile (B). Data represents mean ± SD (n=4).
Figure 5: Chromatogram showing the (abundant) presence of 1-OH midazolam glucuronide in perfusate, 240 min after starting the portal administration of midazolam.
Figure 6: Total production of midazolam 1-OH glucuronide in perfusate during 240 min of continuous administration of midazolam.

### Examples

### Example 1 Comparison of human and porcine liver

### Materials and methods

To study transporter and enzyme expression in porcine liver tissue compared to the expression in human liver tissue, from five healthy domestic pigs (*Sus scrofa domesticus,* 2 male and 3 female, age 10-14 weeks and body weight between 15 and 25 kg) was collected. These animals were additionally used for educational purposes at the Utrecht University (Utrecht, The Netherlands). The local animal welfare office approved the use of these animals for these purposes which was in full compliance with the aim to contribute to the reduction, refinement, and replacement of animal experiments. Before termination, pigs had free access to food and water. Liver tissue of domestic pigs was collected only when defined healthy as judged by a veterinarian.

Human liver samples derived from 15 individuals, of which 5 were anonymously collected at the University Medical center of Groningen (UMCG, Groningen, The Netherlands) and were kindly provided by Prof. Dr. G.M.M. Groothuis (University of Groningen, The Netherlands) and 10 were collected at the Department of Surgery, Uppsala University Hospital (Uppsala, Sweden) and were kindly provided by Prof. Dr. P. Artursson (7 male and 3 female donors; human liver specimens also used in previously published study (Wegler et al., 2017). Collection of redundant tissue from surgeries (collected as waste material) was approved by the Medical Ethical Committee of the UMCG or the Uppsala Ethical Review Board (ethical approval no. 2009/028 and 2011/037). No clinically relevant or identifiable information from the patients was collected

To determine the protein levels of BCRP, BSEP, MDR1, MRP1, MRP2, OATP2B1, OCT1, GLUT1, MCT1, MRP3, NTCP, OATP1B1, OATP1B3 in porcine and human liver tissue, we followed the protocol of membrane isolation and trypsin digestion as previously described for tissue samples and cell lines (Bosgra et al., 2014; Vaessen et al., 2017). All samples were processed in duplicate. LC-MS/MS settings were as previously described (Vaessen et al., 2017). For each peptide 3 transitions were chosen (Q3-1, Q3-2, and Q3-3) for quantitation and confirmation (Table S1). In case no suitable prototypic peptide could be selected for the human and porcine transporter proteins, two separate peptides were selected and synthesized (Table 1). Peptides labeled with 15N and 13C (AQUA peptide) were synthesized (Sigma Aldrich Chemie, Steinheim, Germany) and used as an internal standard for quantification. For each peptide, a calibration curve of 0.01-50 ng/ml and quality controls were included in every run. Data are expressed as fmol transporter protein/ mg liver.

**Table 1. Multiple reaction monitoring (MRM) transitions of the various peptides and the corresponding internal standard (AQUA) used. The peptide sequences were chosen according to the in silico peptide criteria defined by Kamiie et al. (Kamiie et al. 2008) and are exclusively present in the selected protein of interest.**

| ***Name*** | ***Labelled*** | ***Peptide sequence^{a}*** | ***MW*** | ***Q1*** | ***Q3-1*** | ***Q3-2*** | ***Q3-3*** |
|---|---|---|---|---|---|---|---|
| BCRP | unlabelled | SSLLDVLAAR | 1.044.2 | 522.8 | 644.3 | 757.5 | 529.4 |
| | AQUA | SSLLDVLAAR | 1.060.2 | 526.3 | 651.3 | | |
| BSEP | unlabelled | STALQLIQR | 1.029.2 | 515.3 | 657.4 | 841.6 | 529.4 |
| | AQUA | STALQLIQR | 1.045.2 | 518.8 | 664.3 | | |
| GLUT-1 | unlabelled | VTILELFR | 990.2 | 495.8 | 790.5 | 677.4 | 201.2 |
| | AQUA | VTILELFR | 1.00.2 | 500.8 | 800.5 | | |
| MCT-1 | unlabelled | SITVFFK | 841.0 | 421.2 | 173.3 | 641.3 | 201.1 |
| | AQUA | SITVFFK | 851.0 | 426.2 | 651.3 | | |
| MDR1 | unlabelled | AGAVAEEVLAAIR | 1269.5 | 467.7 | 719.4 | 216.1 | 618.4 |
| | AQUA | AGAVAEEVLAAIR | 1276.5 | 471.2 | 726.5 | | |
| MRP-1 | unlabelled | TPSGNLVNR | 957.1 | 479.2 | 428.8 | 759.4 | 672.4 |
| | AQUA | TPSGNLVNR | 973.1 | 482.7 | 432.3 | | |
| MRP2 | unlabelled | VLGPNGLLK | 910.1 | 455.8 | 698.5 | 185.3 | 213.3 |
| | AQUA | VLGPNGLLK | 926.1 | 459.2 | 705.4 | | |
| MRP3 | unlabelled | ALVITNSVK | 944.1 | 472.8 | 760.4 | 661.4 | 548.4 |
| | AQUA | ALVITNSVK | 950.1 | 475.8 | 766.5 | | |
| NTCP-pig | unlabelled | GIYDGTLK | 866.0 | 433.7 | 696.3 | 143.2 | 171.2 |
| | AQUA | GIYDGTLK | 882.0 | 437.2 | 703.4 | | |
| NTCP-human | unlabelled | GIYDGDLK | 880.0 | 440.7 | 710.3 | 143.2 | 171.2 |
| | AQUA | GIYDGDLK | 896.0 | 444.2 | 717.3 | | |
| OATP-1B1 | unlabelled | LNTVGIAK | 815.0 | 408.2 | 399.4 | 588.3 | 288.2 |
| | AQUA | LNTVGIAK | 831.0 | 411.7 | 402.9 | | |
| OATP-1B3 | unlabelled | IYNSVFFGR | 1.102.3 | 551.8 | 826.5 | 249.1 | 526.2 |
| | AQUA | IYNSVFFGR | 1.112.3 | 556.8 | 836.4 | | |
| OATP-1B4-pig | unlabelled | LTLVGIAK | 816.0 | 408.2 | 399.4 | 588.3 | 288.2 |
| | AQUA | LTLVGIAK | 832.0 | 411.7 | 402.9 | | |
| OATP-2B1 | unlabelled | SSISTVEK | 849.9 | 425.7 | 563.3 | 676.3 | 175.1 |
| | AQUA | SSISTVEK | 855.9 | 428.7 | 569.3 | | |
| OCT-1 | unlabelled | LPPADLK | 752.9 | 377.2 | 543.3 | 183.3 | 260.3 |
| | AQUA | LPPADLK | 768.9 | 380.7 | 550.4 | | |

### Results

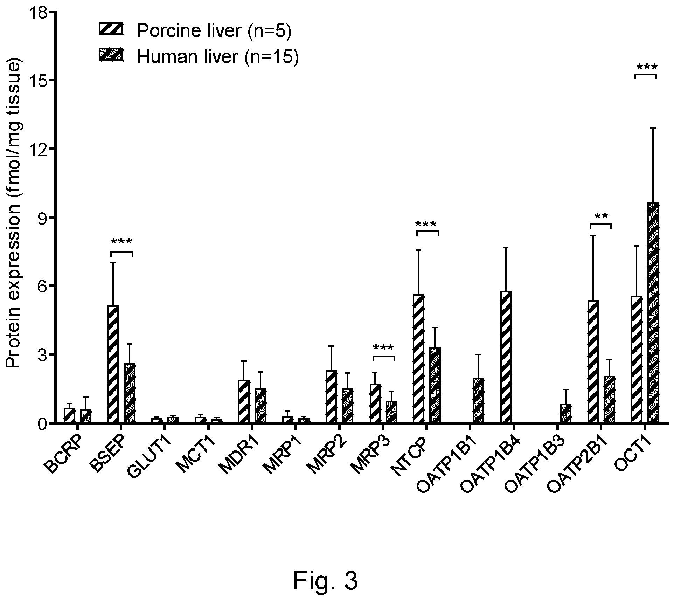

Figure 3 show the comparable transporter and enzyme expression in human and porcine liver.

The LCMS based quantitative proteomics analysis demonstrate:
- comparable expression levels of the main hepatic transporters: MDR1, MRP1, MRP2, MRP3, NTCP, GLUT1 and MCT1
- expression of OCT1 was approximately 1.8-fold higher in human liver compared to porcine livers
- expression of BCRP, BSEP and OATP2B1 was ~2-fold higher in porcine livers than in human livers.

### Example 2 Atorvastatin perfusion

### Materials and methods

**Chemicals.** Atorvastatin was purchased from Bio-Connect (Huissen, The Netherlands). Heparin, taurocholate, and insulin were purchased from Sigma-Aldrich Chemie B.V. (Zwijndrecht, The Netherlands). Atorvastatin lactone, 2-hydroxy atorvastatin, 2-hydroxy atorvastatin lactone, 4-hydroxy atorvastatin, and 4-hydroxy atorvastatin lactone were obtained from Toronto Research Chemicals (Toronto, ON, Canada). Epoprostenol was purchased from R&D Systems (Minneapolis, MN). Vitamin solution, L-glutamine, Minimum Essential Medium (MEM) essential acids, and glutamax were obtained from Gibco (Paisley, Scotland). Calcium gluconate 10% was obtained from Pharmamarket (Hove, Belgium).

**Porcine Livers.** Livers were obtained from a local slaughterhouse (Sus scrofa domesticus, approximately at the age of 6 months, with body weight between 100 and 120 kg). Pigs were sacrificed by a standardized procedure of electrocution followed by exsanguination. Thereafter, 3 L of blood was collected in a container supplemented with 25,000 IU of heparin. All abdominal organs were dissected outside the animal and collected. Within 20 minutes after termination, the vena porta was cannulated and directly flushed by gravity with 3 L of NaCl 0.9% (Baxter BV, Utrecht, The Netherlands) supplemented with 5000 IU of heparin followed by 2 L of ice-cold histidine-tryptophan-ketoglutarate solution (Plegistore, Warszawa, Poland). In the meantime, the arteria hepatica was dissected, cannulated, and subsequently flushed with histidine-tryptophan-ketoglutarate. At the laboratory, side branches were ligated, and the common bile duct was cannulated, whereas the ductus cysticus, derived from the gall bladder, was ligated.

**Normothermic Machine Perfusion.** The porcine livers were perfused using the LiverAssist device (XVIVO, Groningen, The Netherlands). The machine consists of two rotary pumps that provide a pulsatile flow to the hepatic artery and a continuous flow to the portal vein. The system was filled with 2 L perfusion fluid containing red blood cells and perfusate (Table 2). Insulin, taurocholate, heparin, and epoprostenol were provided as continuous infusion at a rate of 10 U/h, 1041 U/h, 10 ml/h (2% w/v), and 8 mg/h, respectively, to maintain liver functioning including bile flow. Additionally, amino acids and vitamins were continuously provided to keep the liver metabolically active. Gas delivery to the LiverAssist consisted of 95% oxygen and 5% carbon dioxide at 2 L/min, and the temperature was set at 39C (body temperature pigs). The livers were perfused with a portal pressure of 11 mmHg and a mean arterial pressure of 50 mmHg. Upon perfusion, additional boluses of sodium bicarbonate and glucose were applied depending on perfusate pH (range 7.35- 7.45) and glucose concentration (>5 mmol/l). Arterial blood gas samples were taken hourly to monitor liver viability (pH, glucose, Na, K, lactate, etc.) using the i-STAT clinical analyzer (Abbot Point of Care Inc., Princeton, NJ).

### Drug administration during perfusion

The portal doses for atorvastatin applied to the system was based on SimCyp calculations. After, pilot experiments, the doses increased 2x for atorvastatin in order to facilitate proper detection by LCMS. After a stabilization period of 120 min, the statin was administered as a slow bolus at a rate of 1mL/min during 10 min to the portal vein. Time of starting the slow bolus was set at t=0 min. Subsequently, perfusate and bile samples were taken for the following 120 min. Arterial blood samples were taken at t=0, 2, 4, 6, 8, 10, 15, 20, 30, 40, 50, 60, 90 and 120 min. Portal samples were taken during the administration of the drug at t=5 and t=10 min to determine the first pass effect. Bile samples were collected in 10 minute fractions. Blood samples were centrifuged directly after collection at 1.3 *g* for 10 min at 4°C and thereafter perfusate (and bile) samples were immediately stored at ≤-70°C until further processed. Drug concentrations in perfusate, bile and liver biopsies were determined by LC-MS/MS analysis as described below.

### Bioanalysis

The concentration of atorvastatin and its metabolites in perfusate and bile was quantified using LC/MS. Briefly, 20 µL of sample was extracted by adding 100 µL of acetonitrile containing internal standard. Samples were vortexed, centrifuged at 3000 rpm for 5 minutes and 100 µL of supernatant was collected in a clean sample plate. Samples were then mixed with 50µL of water, vortexed and injected in to LC/MS for quantification. The details of LC/MS conditions used for the analysis of each compound are shown in table 3. The mass spectrometer (AB Sciex API 5500) was operated in electrospray positive ion mode with the capillary voltage of 5.5 kV and Spray temperature of 550 °C. The multiple reaction monitoring transitions used for all the compounds are shown table 4.

**Table 2 perfusate composition**

| **Components** | **Quantity** |
|---|---|
| Red blood cells | 1000 mL |
| Plasma | 1000 mL |
| Calcium gluconate (10%) | 10 mL |
| Sodium bicarbonate 8.4% solution | To pH of 7.4 |
| Heparin | 1000 IU |
| | |
| Fast-acting insulin | **Continuous infusion** |
| | (10 U/mL; 1mL/h) |
| Taurocholate | **Continuous infusion** |
| | (2% w/v; 10 mL/h) |
| Epoprostenol | **Continuous infusion** |
| | (80 µg in 100 mL; 10mL/h) |
| Heparin | **Continuous infusion** |
| | 1041 U/h (1mL/h) |
| | **Continuous infusion** |
| Vitamin solution, | (1 mL/hr) |
| L-glutamine, | (1 mL/hr) |
| MEM essential acids and | (2 mL/hr) |
| Glutamax | (1 mL/hr) |

**Table 3. Details of the LC/MS conditions used for the analysis of atorvastatin and atorvastatin metabolites.**

| **Compound** | **Column** | **Mobile Phase** | | **Time (sec)** | **Mobile Phase B (%)** | **Flow (ml/min)** |
|---|---|---|---|---|---|---|
| | | **A** | **B** | | | |
| Atorvastatin, atorvastatin lactone, 2-hydroxy atorvastatin, 4-hydroxy-atorvastatin, 4-hydroxy atorvastatin lactone | Macmod; ACE 3 C18-AR; 30×2.1 mm | 0.1% Formic Acid in 95:5 | 0.1% Formic Acid in 50:50 | 15 (Step) | 45 | |
| | | | | 60 (Ramp) | 95 | 0.800 |
| | | Water:Acetonitrile | Acetonitrile:Methanol | 5 (Ramp) | 95 | |
| | | | | 30 (Step) | 95 | |

**Table 4 The Multiple Reaction Monitoring Transition (MRM) of Compounds**

| **Compound** | **MRM Transition (m/z)** |
|---|---|
| Atorvastatin | 559.30→466.00 |
| Atorvastatin lactone | 541.30→448.10 |
| 2-hydroxy atorvastatin | 575.40→440.00 |
| 2-hydroxy atorvastatin lactone | 557.30→448.10 |
| 4-hydroxy atorvastatin | 575.30→440.20 |
| 4-hydroxy atorvastatin lactone | 557.40→448.10 |
| Glyburide (internal standard) | 494.20→369.10 |
| Carbamazepine (internal standard) | 237.10→94.10 |
| Chrysin (internal standard) | 255.10→153.00 |

### Results

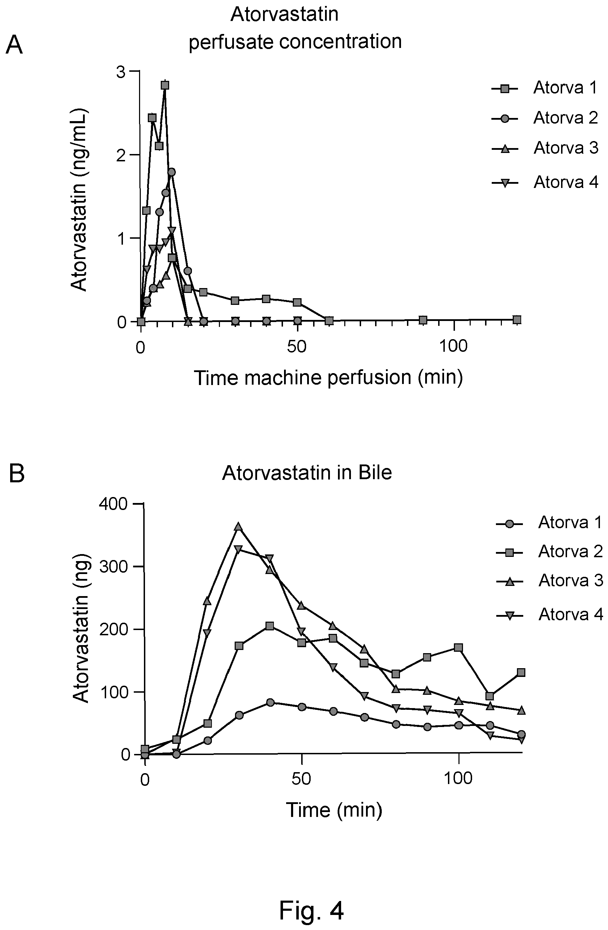

Figure 4 show the plasma and biliary profile of atorvastatin. Within 10 minutes after the start of the bolus administration, atorvastatin was detected in the bile with a Tₘₐₓ of 35.0±5.0 min. A total of 13.0±5.6% of the parent compound was secreted into the bile within 120 min of perfusion.

Atorvastatin is subjected to CYP3A4 mediated hepatic metabolism. We investigated the presence of its known metabolites (atorvastatin lactone, 2-OH atorvastatin, 2-OH atorvastatin lactone, 4-OH atorvastatin and 4-OH atorvastatin lactone) in plasma and bile samples obtained from the perfused livers exposed to atorvastatin (Table 5). All five clinically known metabolites were detected in the bile, but no metabolites were detected in plasma.

**Table 5 Atorvastatin metabolite excretion into the bile (n = 1) upon dosing atorvastatin (0.104 mg) to the portal vein of perfused porcine liver before (0-120 minutes). Metabolite abundance is expressed as percentage of given Atorvastatin dose (0.104 mg)**

| **% biliary clearance** | **0 - 120 min Atorva alone** |
|---|---|
| **Atorvastatin lactone** | **9.17** |
| **2-hydroxy atorva** | **5.33** |
| **2-hydroxy atorva lactone** | **3.48** |
| **4-hydroxy atorva** | **10.28** |
| **4-hydroxy atorva lactone** | **0.400** |

### Example 3 Midazolam

### Materials and methods

Porcine livers were perfused according to the settings of example 2.

### Drug administration during perfusion

A stock solution for midazolam of 1.5 mg/mL was made. After a stabilization period of 60 min, midazolam was administered continuously to the portal vein for a period of 4h with an hourly increase of the midazolam doses (Table 6). Subsequently, perfusate and bile samples were taken for the following 240 min of perfusion. Arterial blood samples were taken at every half hour. Bile samples were collected in 30 minute fractions. Blood samples were centrifuged directly after collection at 1.3 *g* for 10 min at 4°C and thereafter perfusate (and bile) samples were immediately stored at ≤-70°C until further processed. Drug concentrations in perfusate, bile and liver biopsies were determined by UPLC

### Bioanalysis

The concentration of midazolam in perfusate and bile was quantified using UPLC/MS. Briefly, 200 uL of perfusate was extracted by adding 1mL of acetonitrile. Samples were vortexed, centrifuged at 3000 rpm for 5 minutes and 1200 uL of supernatant was collected in a clean sample plate. Samples were dried under nitrogen and subsequently dissolved in 50 µl 10% acetonitrile + 0.1% formic acid in water. 10 uL of bile samples were diluted 500x in 10%ACN + 0,1%FA. 20ul 50%ACN was added to 80 uL of diluted sample. Samples were measured according to settings below.

**LC-MS method**

| | |
|---|---|
| UPLC system | Acquity H-Class (Waters) |
| Mass spectrometer | Q-Exactive Orbitrap HRMS (Thermo) |
| Column | Acquity UPLC BEH C18 (2.1x50 mm, 1.7 µm) |
| Column temperature | 25°C |
| Wash solvent | Acetonitrile/water (90/10; v/v) |
| Sample manager temp | 10°C |
| Mobile Phase 1 (A) | 0.1% formic acid in water |
| Mobile Phase 2 (B) | 0.1% formic acid in acetonitrile |
| Flow rate | 0.6 mL/min |
| Detection | (PRM) in positive ion mode |
| Injection volume | 10 µL |

### Gradient

| **Time** | **Flow** | **% A** | **% B** | **Curve** |
|---|---|---|---|---|
| **0** | 0.6 | 85 | 15 | 6 |
| **0.2** | 0.6 | 85 | 15 | 6 |
| **1.0** | 0.6 | 50 | 50 | 6 |
| **1.5** | 0.6 | 5 | 95 | 6 |
| **2.2** | 0.6 | 5 | 95 | 6 |
| **2.3** | 0.6 | 85 | 15 | 6 |
| **3.2** | 0.6 | 85 | 15 | 6 |

**Table 6 Midazolam dosing perfusion set-up.**

| **Perfusion time** | |
|---|---|
| 0-60 | Start normothermic machine perfusion |
| 60 - 120 | Start dosing midazolam 4.95 mg/hr |
| 120 - 180 | Increase midazolam dosing to 9.90 mg/hr |
| 180 - 240 | Start dosing midazolam 15.00 mg/hr |
| 240 - 300 | Start dosing midazolam 19.95 mg/hr |

### Results

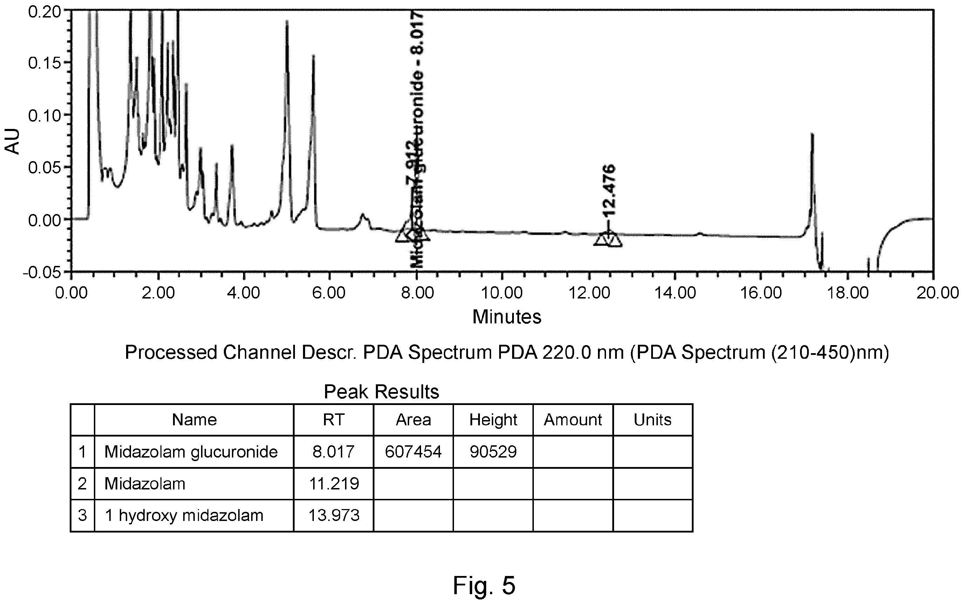

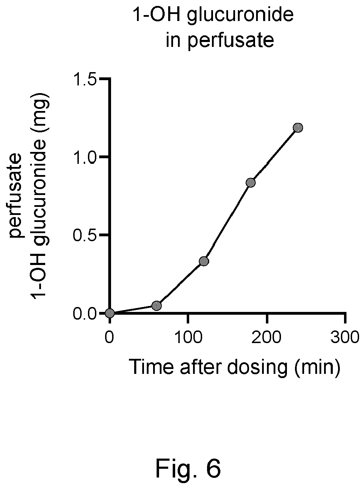

Figure 5 and 6 show the 1-OH midazolam glucuronide formation during 240 min of perfusion. According to table 6, the doses of midazolam administered to the portal vein was increased in time and also an increase in metabolite formation was shown.

Table 7 shows the total metabolite production derived from the different matrices during perfusion. As can be observed in table 7, a high concentration of 1-OH midazolam glucuronide is present in the tissue (1.891 mg). In the perfusate 1.19 mg was measured and the biliary excretion a total of 0.254 mg 1-OH glucuronide was excreted.

**Table 7 Total yield of 1-OH midazolam glucuronide during 240 min of perfusion with an input of 49.8 mg midazolam.**

| **Matrix** | **Yield 1-OH midazolam glucuronide** |
|---|---|
| Perfusate | 1.19 mg |
| Bile | 0.254 mg |
| Tissue | 1.891 mg |
| **Total** | **3.33 mg 1-OH midazolam glucuronide** |

### References

Bosgra S, van de Steeg E, Vlaming ML, Verhoeckx KC, Huisman MT, Verwei M, and Wortelboer HM (2014) Predicting carrier-mediated hepatic disposition of rosuvastatin in man by scaling from individual transfected cell-lines in vitro using absolute transporter protein quantification and PBPK modeling. Eur J Pharm Sci 65:156-166
Kamiie, J., Ohtsuki, S., Iwase, R., Ohmine, K., Katsukura, Y., Yanai, K., ... & Terasaki, T. (2008). Quantitative atlas of membrane transporter proteins: development and application of a highly sensitive simultaneous LC/MS/MS method combined with novel in-silico peptide selection criteria. Pharmaceutical research, 25(6), 1469-1483.
Stevens, L. J., Donkers, J. M., Dubbeld, J., Vaes, W. H., Knibbe, C. A., Alwayn, I. P., & van de Steeg, E. (2020). Towards human ex vivo organ perfusion models to elucidate drug pharmacokinetics in health and disease. Drug metabolism reviews, 52(3), 438-454.
Vaessen SF, van Lipzig MM, Pieters RH, Krul CA, Wortelboer HM, and van de Steeg E (2017) Regional expression levels of drug transporters and metabolizing enzymes along the pig and human intestinal tract and comparison with Caco-2 cells. Drug Metab Dispos 45:353-360.
Wegler C, Gaugaz FZ, Andersson TB, Wisniewski JR, Busch D, Gr€oer C, Oswald S, Noren A, Weiss F, Hammer HS, et al. (2017) Variability in mass spectrometry-based quantification of clinically relevant drug transporters and drug metabolizing enzymes. Mol Pharm 14:3142-3151.

## Claims

1. A method for producing one or more compounds of interest, the method comprising:
- *ex vivo* perfusing a liver or liver tissue and optionally a kidney or kidney tissue with a perfusion liquid comprising a precursor of said one or more compounds of interest, wherein said precursor is a compound that is metabolized in the liver and/or kidney, and
- purifying said one or more compounds of interest from the perfusate, bile, urine, perfused liver or liver tissue and/or perfused kidney or kidney tissue.

2. The method according to claim 1, comprising isolating and purifying the one or more compounds of interest from the perfusate, bile, urine and/or perfused liver or liver tissue.

3. The method according to any one of the preceding claims wherein the liver or liver tissue is continuously perfused with said perfusion liquid.

4. The method according to any one of the preceding claims wherein the liver or liver tissue comprises a portal vein and a hepatic artery and optionally the kidney or kidney tissue comprises a renal artery, and the liver or liver tissue is perfused through the portal vein and the hepatic artery, and optionally the kidney or kidney tissue which is perfused through the renal artery.

5. The method according to any one of the preceding claims wherein the perfusion liquid comprises at least 10 mg, preferable at least 100 mg of said precursor.

6. The method according to any one of the preceding claims wherein the perfusion is performed under normothermic or subnormothermic conditions.

7. The method according to any one of the preceding claims wherein the perfusion liquid is continuously oxygenated.

8. The method according to any one of the preceding claims wherein the perfusion liquid comprises red blood cells and plasma.

9. The method according to any one of the preceding claims wherein the perfusion liquid comprises one or more compounds selected from the group consisting of bile acid, such as cholate or taurocholate, insulin, epoprostenol, heparin, amino acids, vitamins and glucose .

10. The method according to any one of the preceding claims wherein the liver or liver tissue is perfused for at least 4 hours, preferably for 4 to 24 hours.

11. The method according to any one of the preceding claims wherein the liver or liver tissue is comprised in an organ system further comprising a gall bladder or gall bladder tissue and the one or more compounds of interest are further purified from bile, preferably isolated and purified from bile, and/or the liver or liver tissue is comprised in an organ system further comprising a kidney or kidney tissue and the and one or more compounds of interest are further purified from urine, preferably isolated and purified from urine.

12. The method according to any one of the preceding claims wherein the liver or liver tissue and kidney or kidney tissue are perfused in series, preferably wherein the liver or livers tissue is first perfused with the perfusion liquid comprising a precursor of said one or more compounds of interest and the kidney or kidney tissue is perfused with the perfusate from the perfused liver or liver tissue.

13. The method according to any one of the preceding claims wherein the liver or liver tissue is a porcine liver or porcine liver tissue and optionally the gall bladder or gall bladder tissue is a porcine gall bladder or porcine gall bladder tissue and/or the kidney or kidney tissue is a porcine kidney or porcine kidney tissue.

14. The method according to any one of the preceding claims wherein said purifying comprises one or more selected from the group consisting of extraction, including differential extraction, crystallization, distillation, including simple distillation, fractional distillation and steam distillation, and chromatography, including adsorption chromatography, column chromatography, thin layer chromatography (TLC), partition chromatography and combinations thereof, preferably wherein the purifying comprises extraction using an organic solvent.

15. The method according to any one of the preceding claims further comprising packaging the one or more purified compounds of interest.
